# EUROPÄISCHE PATENTSCHRIFT

(11) **EP 0 773 914 B1**
(45) Veröffentlichungstag und Bekanntmachung des Hinweises auf die Patenterteilung: **14.10.1998**
(21) Anmeldenummer: 95927626.2
(22) Anmeldetag: 01.08.1995
(51) Int. Cl.: C05F 17/02, C05F 17/00, C05F 3/00, C05F 9/02, B01F 7/08, B30B 9/18

(54) **VERFAHREN UND VORRICHTUNG ZUR BEHANDLUNG VON ORGANISCHEN BIO-RESTSTOFFEN**
PROCESS AND DEVICE FOR TREATING ORGANIC BIOLOGICAL RESIDUES
PROCEDE ET DISPOSITIF DE TRAITEMENT DE RESIDUS ORGANIQUES BIOLOGIQUES

(30) Priorität: 04.08.1994 DE 4427644
(43) Veröffentlichungstag der Anmeldung: 21.05.1997
(73) Patentinhaber: Maschinenfabrik Ernst Hese GmbH, 45881 Gelsenkirchen (DE)
(72) Erfinder: DERTMANN, Volkmar, D-45657 Recklinghausen (DE); ZILLMANN, Sven-Olaf, Friedrich, D-46145 Oberhausen (DE)
(74) Vertreter: Vomberg, Friedhelm, Dipl.-Phys.
(86) Internationale Anmeldenummer: DE9501018
(87) Internationale Veröffentlichungsnummer: WO9604220

(56) Entgegenhaltungen:
- EP-A- 0 536 767
- WO-A-94/24071
- BE-A- 723 768
- FR-A- 2 541 669
- US-A- 5 207 904

## Beschreibung

Die Erfindung betrifft ein Verfahren zur Behandlung von organischen Bio-Reststoffen, insbesondere aus kommunalen und/oder gewerblichen Abfallstoffen einschließlich roher und/oder gekochter Speisereste, landwirtschaftlicher Abfälle, insbesondere Tierexkremente und/oder pflanzlicher Bestandteile, die unter intensivem Mischen und Homogeniesieren in eine flüssige Fraktion mit in Wasser gelösten oder lösbaren C-haltigen organischen Bestandteilen (DOC) von einer aus den verbleibenden Feststoffen bestehenden festen Fraktion durch Pressen getrennt werden.
Ein solches Verfahren ist aus der US-A-5 207 904 bekannt.

Die Erfindung betrifft ferner eine Vorrichtung zur Mischung und Homogenisierung von Bio-Reststoffen vor dem Abpressen der Flüssigfraktion von der Festfraktion, mit einem mindestens zwei parallele Förderschnecke enthaltenden Behälter, unter Bildung eines geschlossenen Förderkreislaufes.
Eine derartige Vorrichtung beschreibt die EP 0 536 767 A1.

Unter Bio-Reststoffen werden neben den bereits genannten Abfallstoffen alle Stoffe verstanden, die einen der Vergärung zugänglichen Gehalt an Kohlenstoff in gelöster Form (DOC) aufweisen. Dies können beispielsweise pflanzliche Grünabfälle, wie Langgras, Laub, Rasenschnitt, Astwerk, Gehölzschnitt, Blumen, Ernterückstände aus dem Garten/Landschaftsbau, aber auch Abfälle aus der gemüse- und obstverarbeitenden Industrie sein, wie Schalen von Früchten, Ölfrüchten, Trester, Bierschlämme, Hefen, Kokosfasern, Blätter und Strünke aus der Konservenindustrie, Kartoffelschalen und Blattrückstände, Rinden und Späne aus der Holz- und Papierindustrie, organischer kommunaler oder gewerblicher Stadtmüll und schließlich landwirtschaftliche Abfälle.

Seit langem ist es bekannt, reine Grünabfälle zu kompostieren, wozu die zerkleinerten Abfälle mit oder ohne Zugabe von Kompostzuschlägen in Kompostmieten aufgesetzt und einem Verrottungsprozeß überlassen werden, bei dem die Abfälle allmählich zersetzen. Es ist inzwischen auch bekannt, daß die Verrottungszeit durch Umsetzen der aufgeschichteten Mieten sowie eine gezielte Prozeßführung durch Überwachung der Temperatur und/oder Mietenfeuchtigkeit beschleunigt werden kann.

In der EP 0 429 744 A2 wird ein Verfahren beschrieben, bei dem das Abfallmaterial nach Vorbereitung durch Sieben, Trennen und Zerkleinern einem Gärbehälter zugeführt wird und zum Zwecke der Einleitung und Aufrechterhaltung eines aeroben biologischen Zersetzungsprozesses unter ständigem Rühren allmählich gegen die zentrale Austrittsöffnung des Gärbehälters verschoben wird. Zur Verbesserung dieser Fermentiermethode wird eine aerobe biologische Verrottung biologischer Abfälle vorgeschlagen, bei der die Abfälle unter ständigem Rühren innig mit Luft vermischt und dadurch einer bakteriellen aeroben Zersetzung zugeführt werden und das aus dem Verrottungsprozeß hervorgegangene Produkt dem Aufnahmebehälter entnommen und einer Veredelung unterzogen wird. Hierzu soll der Aufnahmebehälter etwa bis zur Hälfte mit festem Abfall und anschließender Zugabe flüssiger Substanzen gefüllt und während einer Verweilzeit von 2 bis 3 Stunden umgerührt werden, bevor dekantierter Klärschlamm sowie Weißkalk zugegeben wird und das Umrühren bis zu einer Verweilzeit von 69 oder 70 Stunden fortgesetzt wird.

Besondere Probleme ergeben sich insbesondere im Bereich der ersten Verfahrensstufe bzw. dem betreffenden Mischbehälter, da die Gefahr einer Bio-Reststoff-Brückenbildung mit schließlicher Verstopfung des Mischbehälters oder zumindest einer unzureichenden Homogenisierung vermieden werden müssen.

Es ist daher Aufgabe der vorliegenden Erfindung ein Verfahren und eine Vorrichtung anzugeben, bei denen eine Brückenbildung vermieden wird und das aufgegebene Material optimal homogenisiert wird.

Diese Aufgabe wird zum einen durch das Verfahren nach Anspruch 1 gelöst, das erfindungsgemäß dadurch gekennzeichnet ist, daß die organischen Bio-Reststoffe vor dem Pressen über oberhalb und unterhalb eines schrägliegenden Tisches oder Tischbleches jeweils parallel hierzu angeordnete Förderschnecken in einem geschlossenen Kreislauf geführt werden, in dessen Verlauf die Bio-Reststoffe aufgelockert und die aufgelockerten Bio-Reststoffe befeuchtet und/oder belüftet werden, wobei die Auflockerung der Bio-Reststoffe durch eine Erhöhung der Fördergeschwindigkeit in einem Teil des Kreislaufes durchgeführt wird. Insbesondere hat sich herausgestellt, daß es nicht ausreicht, wenn eine Schnecke oder zwei parallel angeordnete in derselben Förderrichtung fördernde Schnecken die Bio-Reststoffe in einer Aufwärtsbewegung fördern, die dann in einem freien Teil des Behälters mit den Förderschnecken herabfallen sollen. Je nach Ausgangsmaterial und Restfeuchte kommt es zu einer Bio-Reststoff-Brückenbildung, die schließlich in eine totale Verstopfung des Mischbehälters mündet. Wird hingegen durch die Förderschnecken ein geschlossener Kreislauf gebildet, d.h., wird auch die Abwärtsbewegung des aufgegebenen Materials durch eine Förderschnecke unterstützt, so wird eine Brückenbildung wirksam vermieden und gleichzeitig eine bessere Materialdurchmischung erreicht. Dies gilt auch im Hinblick auf einen homogenen Befeuchtungsgrad.

Weitere Verfahrensverbesserungen ergeben sich bei Anwendung der in den Ansprüchen 2 bis 7 beschriebenen Maßnahmen.

Vorzugsweise werden die Fördergeschwindigkeiten über das Steigungsmaß mindestens einer Förderschnecke eingestellt. Bei dieser Ausführungsform kann vorzugsweise eine Schneckenwelle verwendet werden, die mehrere Schneckenwendeln unterschiedlichen Steigungsmaßes besitzt, so daß die Fördergeschwindigkeit nur über das Steigungsmaß bestimmt ist. Dies garantiert wegen des konstanten Verhältnisses der unterschiedlichen Schneckensteigungen entsprechende Verhältnisse der Fördergeschwindigkeiten. Ein Schneckenabschnitt mit größeren Steigungsmaß ist gleichbedeutend mit einer Materialauflockerung durch eine entsprechend höhere Fördergeschwindigkeit.

Nach einer weiteren Ausgestaltung der Erfindung befinden sich oberhalb und unterhalb einer schräggestellten als Tisch oder Tischblech ausgebildeten festen Ebene jeweils mindestens eine Schnecke, die Bio-Reststoffe aufwärts- und abwärtsfördert. Die oberhalb des Tisches oder Tischbleches befindlichen Schnecken laufen gegenüber entsprechenden Schnecken, die sich unterhalb des Tisches oder Tischbleches befinden, gegenläufig. Bereits zwei Fördermittel oder Fördermittelgruppen, bestehend aus Schneckenpaaren oder mehr als drei in derselben Richtung fördernden Förderschnecken, sorgen für einen geschlossenen Förderkreislauf.

Nach einer weiteren Ausgestaltung der Erfindung werden die Bio-Reststoffe über eine oder mehrere unterhalb des Tisches angeordnete Schnecken mit über ihrer Länge unterschiedlichen Wendelsteigungen und nach oben zunehmendem Steigungsmaß gefördert. Vorzugsweise wird das Steigungsmaß über die Länge der Schnecke bzw. Schnecken unterhalb des Tisches progressiv geändert. Die Änderung des Schneckensteigungsmaßes bewirkt ein Auseinanderziehen des Reststoff-Materialstromes und damit eine optimale Auflockerung bzw. Oberflächenvergrößerung, die eine verbesserte Befeuchtung mit anschließender Homogenisierung erzielen läßt.

Um einer Verstopfung von Lüftungseinrichtungen, wie Belüftungsdüsen, entgegenzuwirken, wird vorzugsweise die Belüftung der im Kreislauf geführten Bio-Reststoffe über in Förderrichtung eingeblasene Luft durchgeführt.

Nach hinreichender Durchmischung der Bio-Reststoffe und ihrer Homogenisierung gibt es nach der vorliegenden Erfindung zwei Möglichkeiten zur Trennung der Feststoffraktion von der Flüssigfraktion. Zum einen kann dies in ein und demselben Behälter durch Zuschaltung eines Preßorganes oder nach Abgabe des homogenisierten Gemisches in eine separate Preßeinrichtung durchgeführt werden. Während die erste Variante insbesondere bei kleineren Durchsatzmengen den Vorzug findet, bietet sich die zweite Variante immer dann an, wenn große Mengen von Bio-Reststoffen zu behandeln sind, bei denen eine separate Preßeinrichtung lohnend erscheint. Insbesondere kann hierbei die Möglichkeit genutzt werden, daß parallel mehrere Mischbehälter mit einer einzigen Preßeinrichtung sukzessive hintereinander bedient werden. Nach Abgabe des homogenisierten Gemisches kann unabhängig vom Abpressen des betreffenden Materials die Homogenisierungsvorrichtung wieder neu befüllt werden. Verwendet man nur eine einzige Preßvorrichtung, so wird diese vorzugsweise unter den betreffenden Behälter gefahren oder an ihm angedockt, um das Abströmen von Gasen oder das Überschwappen von Restflüssigkeiten zu vermeiden.

Während des Mischens und Homogenisierens wird vorzugsweise die Ausgangsfeuchte oder eine Feuchte von 70 bis 80 Vol.-%, zumindest jedoch 40 bis 60 Vol.-% eingestellt, da nur bei relativ hohen Feuchten ein Auswaschen der C-haltigen Bestandteile möglich ist. Vorzugsweise wird das Prozeßwasser rezyklisierend in die Misch- und Homogenisierungsstufe geleitet, das nach erfolgter Methanisierung (Vergärung) im Anaerob-Reaktor anfällt. Ggf. kann auch während der Kreislaufführung des Bio-Materials Flüssigkeit abgesogen werden, in der bereits C-haltige Bestandteile durch Auswaschen enthalten sind. Die hierdurch entfernte Flüssigkeit wird in entsprechender Weise durch Befeuchtung ersetzt.

Eine weitere Optimierung der Prozeßsteuerung ergibt sich, wenn eine Temperatur von bis zu 70°C konstant aufrechterhalten wird, ggf. mit der anschließenden Maßnahme, abschließend Wärme zur Erzeugung einer Temperatur von 80°C bis 90°C zuzuführen, um eine möglichst vollständige Hygienisierung durchzuführen. Nicht benötigte Wärme, die bei exotherm verlaufenden Reaktionen im Misch- und Homogenisierungsbehälter entsteht, wird vorzugsweise abgezogen und anderweitig verwendet, z.B. in einem Anaerob-Reaktor.

Nach einer weiteren Ausgestaltung der Erfindung wird erst nach Unterschreiten eines vorgebbaren Gehaltes an gelösten C-haltigen Bestandteilen in der aus der Trennstufe abgeführten Flüssigkeit die in diesen Stufe volumenmäßig zusammengefallene Reststoffmenge auf einen möglichst niedrigen Restfeuchtegehalt abgepreßt, bevor die gesamte Charge an festen Reststoffen abgeführt und anschließend nachkompostiert wird. Die Nachkompostierung kann nach üblichen, nach dem Stand der Technik bekannten Verfahren, mit und ohne Zugabe von Zuschlagstoffen durchgeführt werden.

Die aus der Trennstufe, d.h., nach dem Trennen der festen Fraktion von der flüssigen Fraktion ausgeschleusten Flüssigkeiten liegen als Suspensionen vor. Um einen nachgeschalteten Anaerob-Reaktor für die Flüssigkeit nicht mit Feststoffanteilen bzw. Schlämmen zu belasten, wird die betreffende Suspension vor Einleitung in den Anaerob-Reaktor sedimentiert. Vorzugsweise wird der bei Sudimentieren als Schlamm anfallende Feststoff als Animpfmaterial zur Reaktionsbeschleunigung, weiter vorzugsweise nach Sauerstoffanreicherung in Form von Belebtschlamm nach frischer Füllung der Homogenisierungsstufe den Bio-Reststoffen zugeführt.

Die genannte Aufgabe wird ferner durch die im Anspruch 8 beschriebene Vorrichtung gelöst, die erfindungsgemäß dadurch gekennzeichnet ist, daß oberhalb und unterhalb eines schrägliegenden Tisches oder Tischbleches jeweils parallel hierzu Förderschnecken angeordnet sind, wobei die untere(n) Förderschnecke(n) das Material aufwärts- und die obere(n) Förderschnecke(n) das Material abwärtsfördern, wodurch ein geschlossener Förderkreislauf entsteht. Vorteile dieser Misch- und Homogenisierungsvorrichtung sind bereits vorstehend erörtert worden.

Vorzugsweise Ausgestaltungen der Vorrichtung werden in den Ansprüchen 9 bis 15 beschrieben.

So erzielt man mit einer geringeren Fördergeschwindigkeit, mit der die obere oder oberen Förderschnecke(n) im Vergleich zu der unteren bzw. den unteren Förderschnecken eine Vorverdichtung des Materials am Ende der unteren Förderschnecke bzw. jeder unteren Förderschnecke. Etwa hier abgepreßte Flüssigkeit kann über einen Austrag bzw. Ablauf entnommen oder sonstwie abgesogen werden.

Nach einer weiteren Ausgestaltung ist zumindest eine der Schnecken, vorzugsweise die untere bzw. die unteren Schnecken mit unterschiedlichen Steigungen der Schneckenwendel ausgestattet. Die Änderung des Schneckenwendel-Steigungsmaßes ist nach einer weiteren Ausgestaltung der Erfindung progressiv mit dem Vorzug, daß das Material bei größerem Steigungsmaß aufgelockert wird und in diesem Bereich auch optimal befeuchtet werden kann.

Zum Abpressen der Bio-Reststoffe ist bei einer weiteren Ausgestaltung der Erfindung ein Schieber in den Förderweg bewegbar, gegen den das Material zum Abpressen gefahren wird. Der Öffnungsgrad des Schiebers, nämlich das Querschnittsmaß des blockierten Förderweges, ist vorzugsweise regelbar. Hiermit kann sichergestellt werden, daß bestimmte voreinstellbare Preßdrücke nicht überschritten werden.

Die abgepreßte flüssige Fraktion ist über Lochbleche, die vorzugsweise mit Gewebedraht versehen sind, abführbar. Beispielsweise können Lochbleche mit ca. 35 mm langen und 20 mm breiten Langlöchern versehen werden, die mit einem Gewebetuch einer Maschenweite von 0,5 mm abgedeckt sind.

Nach einer weiteren Ausführungsform der Erfindung sind in verschiedenen Förderbereichen unterschiedliche Drücke einstellbar, insbesondere auch ein Unterdruck zur Wasserabsaugung auch während des Umlaufes zur Homogenisierung und Hygienisierung des Fördermateriales.

Vorzugsweise wird im Bereich eines größeren Förderschneckensteigungsmaßes eine Flüssigkeitseingabevorrichtung angeordnet, damit die Befeuchtung auf das Fördermaterial dann erreicht, wenn dies einen größtmöglichen Lockerungsgrad besitzt.

Nach einer Weiterbildung der Erfindung sind in Förderrichtung gerichtete Düsen zur Luftzufuhr, vorzugsweise oberhalb des Tisches oder Tischbleches vorgesehen. Auch hier wird der Vorteil des aufgelockerten Materiales (ähnlich wie bei der Befeuchtung) genutzt, ferner stellen die geometrischen Anordnungen der Düsen sicher, daß diese weitgehend vor Verstopfungen geschützt sind. In einer bevorzugten Ausführungsform sind im Tisch oder Tischblech in Förderrichtung ansteigende Rampen vorgesehen, an deren vorzugsweise in bezug auf die Tisch(blech)ebene vertikaler Rückseite die Düsenaustrittsöffnungen angeordnet sind. Durch diese Rampen wird das Fördermaterial von der Tischblechbasisebene abgehoben, was einen weiteren Schutz der Düsen bewirkt.

Die vorstehend beschriebene Vorrichtung mit einer integrierten Preßeinrichtung ist immer dann von Vorteil, wenn geringere Mengen von Bio-Reststoffen verarbeitet werden sollen, für die ein Reaktorbehälter mit Förderschnecke ausreichend ist. Bei anfallenden größeren Mengen, bei denen ohnehin mehrere Reaktoren mit Fördereinrichtungen zur Mischung, Homogenisierung und Hygienisierung verwendet werden müssen, ist nach einer Weiterbildung der Erfindung eine separate, vorzugsweise einzige Preßvorrichtung vorgesehen, in die der homogenisierte und hygienisierte Inhalt eines Reaktors zum Pressen entladbar ist. Die einzelnen Homogenisierungsbehälter besitzen hierzu entsprechende wiederverschließbare öffnungsklappen, die nur zum Entleeren in die Preßvorrichtung geöffnet werden. Um die Preßvorrichtung leicht an jeden einzelnen Reaktor zum Mischen und Homogenisieren der Bio-Reststoffe heranführen zu können, ist die Preßvorrichtung auf Schienen oder Rädern verfahrbar. Die Preßvorrichtung enthält vorzugsweise eine oder mehrere Schneckenpressen, die das Material gegen eine Preßwand fördern. Anders als bei den Homogenisierungs-Reaktoren kann die Preßeinrichtung in horizontaler Richtung arbeiten, d.h., die Schneckenpressen können horizontal angeordnet sein.

Sowohl im Falle der kombinierten Misch- und Abpreßvorrichtung in einem Reaktor als auch bei getrennt angeordneter Preßvorrichtung wird nach einer Weiterbildung der Erfindung der Abpreßgrad über einen mit der Preßschnecke verbundenen Stromaufnehmer gemessen. Wie bereits oben im Falle des kombinierten Reaktors erwähnt, kann dieser Stromaufnehmer bei Überschreiten einer einstellbaren Sollwertschwelle die Presse abschalten bzw. die Preßwand unter Freigabe einer Öffnung herunterfahren.

Schließlich ist der Stellwinkel des Behälters zum Mischen und Homogenisieren veränderbar, so daß je nach Materialeigenschaften, wie Dichte, Feuchte etc., der optimale Einstellwinkel gewählt werden kann.

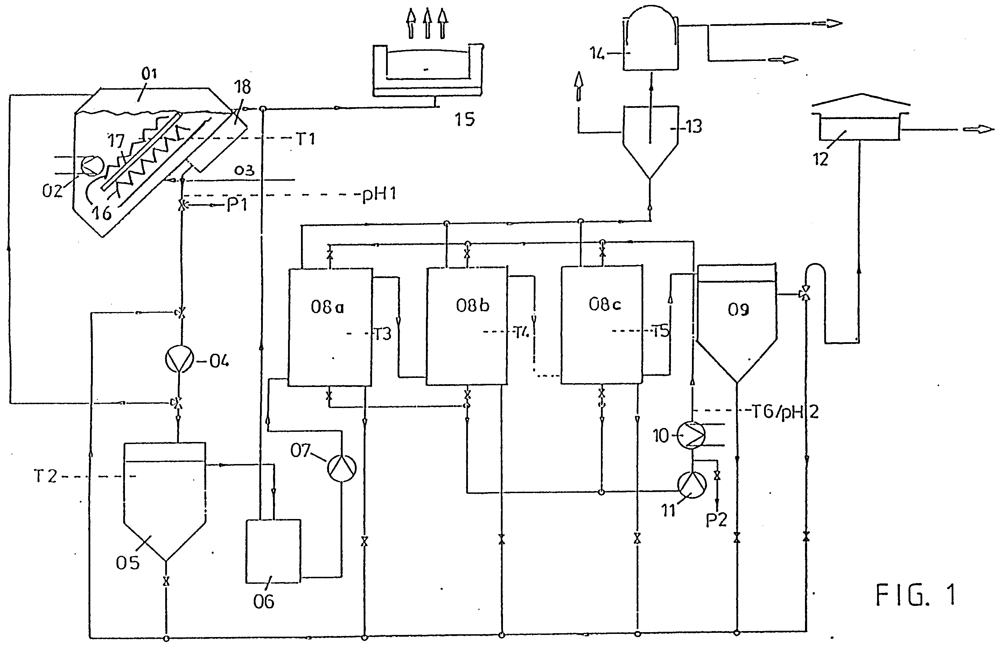

Ausführungsbeispiele der Erfindung sind in den Zeichnungen dargestellt. Es zeigen
- Fig. 1: eine schematische Übersicht einer Anlage zur Kompostierung und Naßvergärung von biologischen Abfällen,
- Fig. 2: eine schematische Seitenansicht der erfindungsgemäßen Vorrichtung zum Mischen, Homogenisieren und Hygienisieren der Bio-Reststoffe (Feststoff-Reaktor),
- Fig. 3: einen Schnitt entlang der Linie III - III nach Fig. 2,
- Fig. 4: eine Schnittansicht entlang der Linie IV - IV in Fig. 2,
- Fig. 5: einen Schnitt im Bereich des verstellbaren Schiebers,
- Fig. 6: eine Schnittansicht entlang der Linie VI -VI nach Fig. 5,
- Fig. 7: einen Längsschnitt durch die unter dem Tischblech liegende Förderschnecke, hier als Förderschneckenpaar und

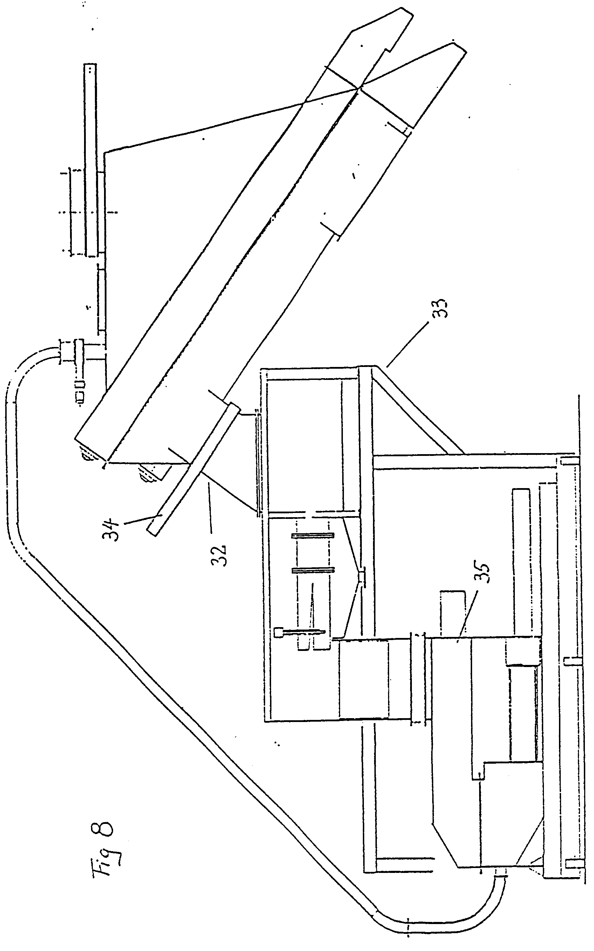

- Fig. 8: eine alternative Ausführungsform eines Homogenisierungs- und Hygienisierungs-Reaktor mit getrennt angeordneter lösbar anschließbarer Presse.

Die erfindungsgemäße Vorrichtung ist Teil einer Anlage, deren wesentliche Teile neben dem als Feststoff-Reaktor 01 ausgebildeten erfindungsgemäßen Vorrichtung noch der aus drei Teilen bestehende Anaerob-Reaktor 08a, b und c ist. Die zu behandelnden biologischen Rest- oder Abfallstoffe werden in den Feststoff-Reaktor 01 gegeben, in dem sie von mehreren Förderschnecken 16 im Kreislauf gefördert und nach Erreichen des gewünschten Homogenisierungs- und Hygienisierungsgrades abgepreßt werden. Die Förderschnecken 16 sind oberhalb und unterhalb eines Tisches bzw. von Tischblechen 17 angeordnet, die als Wärmetauscher 2 ausgebildet sein können. Mit 18 ist eine wiederverschließbare Entleerungsvorrichtung bezeichnet. Durch die Schneckenförderer 16 werden die im Kreislauf umgewälzten Abfallstoffe kontinuierlich ausgewaschen, um die darin enthaltenen Kohlenstoffgehalte gewinnen zu können. Die gesteuerte Sauerstoffzufuhr 03 gewährt eine optimale mikrobielle Aktivität. Die Sauerstoffzufuhr kann ggf. über Sauerstoffgehaltsmessungen oder CO₂-Konzentrationsmessungen gesteuert bzw. geregelt werden. Während der in dem Reaktor 01 stattfindenden aeroben Intensivrotte werden energiereiche polymere Substanzen, wie Fette, Proteine und Kohlenhydrate in energieärmere, monomere Bestanteile, wie Fettsäuren, Aminosäuren und Zucker, zerlegt. Die Dauer der in dem Feststoff-Reaktor 01 stattfindenden vorgeschalteten aeroben Intensivrotte ist variabel und von der Zielsetzung abhängig, ob mehr Biogas oder mehr Kompost produziert werden soll. Beispielsweise können während einer 48-stündigen Aufenthaltszeit im Feststoffreaktor 01 bereits die leicht und mittelschwer abbaubaren organischen Substanzen so weit in niederkettige Fettsäuren und Alkohole gespalten werden, daß sie anaerob im Anaerob-Reaktor 08a, b und c weiter zum hauptsächlichen Endprodukt Methan und Kohlendioxid durch acetogene und methanogene Bakterien abgebaut werden können. Die Fettsäuren und Alkohole sind in bei der Umsetzung austretendem Wasser, das durch die natürliche Feuchte vorhanden ist oder im Zellwasser gebunden und ausgepreßt wird, gelöst und suspendiert. Die Suspension mit hohen Anteilen an aerob hydrolisierten organischen Substanzen wird über das bereits genannte Sieb von der Masse im Feststoffreaktor abgetrennt. Beim Auslaugen der löslichen organischen Bestandteile zusammen mit der aeroben Intensivrotte findet eine starke Volumenreduktion (70 bis 80 Vol-%) des Materials statt. Die aus den biologischen Abfällen gewonnene Suspension wird über ein Ventil P1 durch die Pumpe 04 in einen Sedimentator 05 gefördert. Die dort abgetrennten Feststoffe, die sich als Schlamm niederschlagen, können zum Beimpfen des frisch eingefüllten biologischen Abfalles wieder in den Feststoffreaktor 01 gepumpt werden. Das feststofffreie, mit organischen Inhaltsstoffen, insbesondere Kohlenstoffanteilen, angereicherte Wasser wird in den Anaerob-Reaktor 08 gepumpt. Die restliche, ausgewaschene Feststoffmasse wird als Frischkompost mit einem Rottegrad von I und II einer Nachrotte in Form einer Mietenkompostierung unterworfen. Der Frischkompost wird abgefahren.

Das Befüllen des Feststoffreaktors erfolgt über eine verschließbare Öffnung im Feststoffreaktoroberteil, die nur während des Befüllens geöffnet wird. Das Ausschleusen des Frischkompostes erfolgt über eine elektromotorisch betriebene Austragsschleuse. Das Material fällt in einen verschließbaren Container (nicht dargestellt).

Zur Vermeidung von Geruchsemissionen ist der Feststoffreaktor über eine Gasleitung mit einem Kompostfilter 15 verbunden. Während des Betriebes bleibt der Feststoffreaktor vorzugsweise gasdicht verschlossen. Eingeblasene Luft zur Erzeugung aerober Verhältnisse verläßt den Reaktor nur durch das Kompostfilter. Wird der Reaktor zur Befüllung geöffnet, beginnt ein in der Gasleitung montierter Seitenkanalverdichter automatisch Luft vom Reaktor in das Kompostfilter zu saugen. Es entsteht hierbei eine invers gerichtete Luftströmung, so daß eine Geruchemission aus dem Feststoffreaktor 01 nicht entstehen kann.

Um sicherzustellen, daß das von Feststoffen befreite bzw. die stark feststoffreduzierte Suspension möglichst keinen Sauerstoff in den Anaerob-Reaktor 08 einbringt, ist eine Ultraschallentgasungseinrichtung 06 vorgesehen, durch die die Flüssigkeit geleitet und mittels der Pumpe 7 schließlich in den Anaerob-Reaktor 08 geführt wird. Der mehrstufige Anaerob-Reaktor 08a, b und c, der mittels kommunizierender Röhren im Kreislauf betreibbar ist, ist als Festbettreaktor ausgerüstet. Dem Anaerob-Reaktor 08 ist ein weiterer Sedimentator 09 nachgeschaltet, durch den das noch mit geringen Feststoffanteilen belastete Wasser nach der Methanisierung geführt und von Feststoffen befreit wird. Das Wasser kann anschließend rezyklierend in den Kreislauf zurückgeführt werden. Der Anaerob-Reaktor besitzt einen Wärmetauscher 10, der, vorzugsweise über eine entsprechende Regelung, mit dem Wärmetauscher 02 in Verbindung steht, so daß die bei der exothermen Reaktion im Festbettreaktor 01 anfallende Wärme in den Anaerob-Reaktor 08 geführt werden kann. Die Pumpe 11 unterstützt eine etwa notwendige Kreislaufförderung der Flüssigkeit durch die Stufe 08a, b und c.

Das den Anaerob-Reaktor 08 verlassende Wasser, mit einem DOC-Gehalt von 1 g/l und einem CSB-Gehalt von ca. 2 bis 3 g/l, wird in eine Pflanzenkläranlage 12 geleitet und von dort bis zur Vorfluterqualität gereinigt. Hierbei handelt es sich um eine mit Helophyten besetzte, vertikal durchströmte, mehrschichtige Festbettfilteranlage mit selbsttätigem Intervallbetrieb. Dieser Festbettfilter ist durch die Abfolge von aerober Bodenschicht mit einem Durchlässigkeitsbeiwert (Kf) von 5 x 10⁻³ m/s charakterisiert. In dieser Schicht kommt es zur Nitrifizierung des Stickstoffes und vor allem zur Oxidation der im Wasser gelösten restlichen organischen Substanz. Die darauffolgende dichte Bodenschicht mit einem Kf-Wert von 10⁻⁷ m/s begünstigt eine Denitrifikation des Stickstoffes, welcher gasförmig als N₂ durch eine entsprechende Leitung in die Atmosphäre entweichen kann. Durch den höheren Feinkornanteil wird ebenfalls ein hohes Phosphatbindungspotential sichergestellt. Die Durchlässigkeit der unteren Filterschicht entspricht der oberen. Es kommt zu einer Restmineralisation der organischen Substanz, Nitrifikation vorhandener N-Komponenten und zu einer weiteren Keimzahlreduktion.

Versuche haben erwiesen, daß dieses vertikal durchströmte System der Pflanzenkläranlage, die an Wasser mit Vorfluterqualität gestellten Kriterien einhält, diese sogar unterschreitet.

Über das Wurzelsystem des Schiffes (Rhizom) wird die Sickerleistung ständig gewährleistet. Die Halme beschatten den Bodenkörper und verhindern eine unerwünschte Algenbildung. Sie bilden ein günstiges Mikroklima für Mikroorganismen. Eine wichtige Eigenschaft der Pflanzen besteht in der Fähigkeit, über ein spezielles Luftleitgewebe (Aerenchym) Sauerstoff in den Boden einzutragen. Die Anlage ist auf einer Fläche von 6 m x 5 m untergebracht. Der Wasserabfluß der Anlage wird aufgefangen und über eine Woche gehältert. Erst nach Analyse des Wassers auf die Parameter nach Indirekteinleiterverordnung wird über die Einleitung in die Vorflut oder den Transport in eine Kläranlage entschieden.

Das im Anaerob-Reaktor entstehende Biogas gelangt über einen Gasabscheider 13 in den Gasspeicher 14, der zur Pufferung von Mengenschwankungen bei der Biogaserzeugung dient, um eine gleichmäßige Füllung eines nachgeschalteten Gasmotors aufrechtzuerhalten. Hier kann ein Sterling-Motor eingesetzt werden.

Der Kompostfilter 15 der aus jeder Prozeßstufe gelangende geruchsbeladene Abluftströme aufnehmen kann, besteht vorzugsweise aus einer in 2,5 m-Schachtringen, bis zur Oberkante in den Boden eingelassenen Spezialkompostschicht. Das zu desodorierende Gas wird von der Unterseite her durch das Filter geleitet. Es durchströmt die Kompostschichten. Geruchsaktive Substanzen ad- und absorbieren und werden durch die Mikroorganismen verstoffwechselt. Das den Filter verlassende Gas ist normalerweise annähernd geruchsneutrag oder weist einen leicht erdigen Geruch auf, der jedoch nur direkt oberhalb des Filters wahrnehmbar ist. Die Abluft ist frei von möglichen pathogenen Keimen, die ggf. über den Abfall in den Feststoffreaktor 01 eingetragen werden.

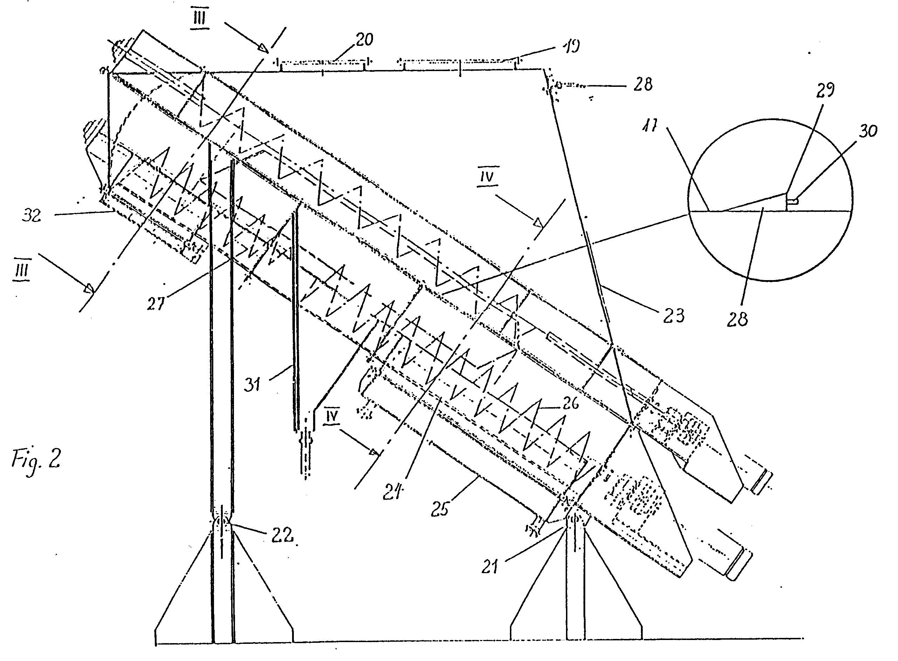

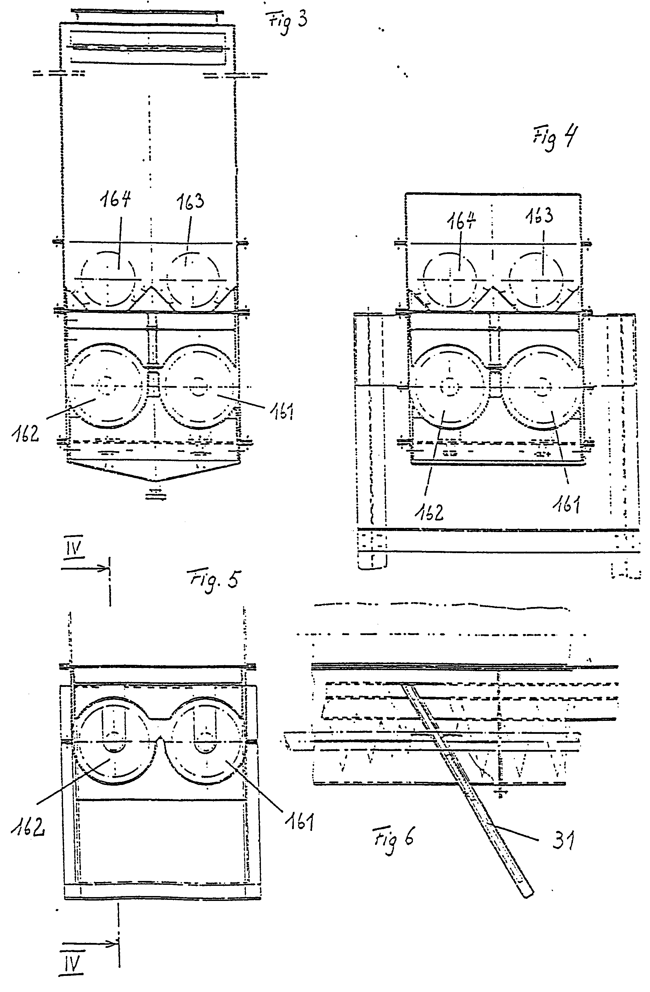

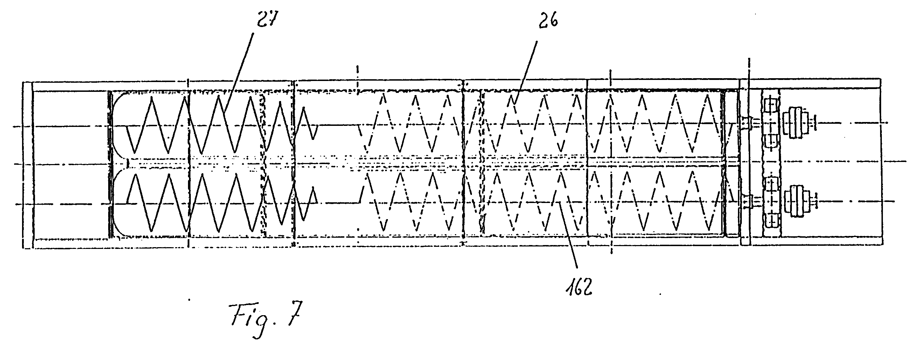

Aus Fig. 2 bis 7 sind Details des Feststoff-Reaktors 01 ersichtlich. Dieser besitzt eine obere Füllöffnung 19 sowie eine weitere wiederverschließbare Öffnung 20 über die das Feststoff-Reaktorinnere vom Wartungspersonal erreichbar ist. Der Reaktor ist über Drehachsen 21 und 22 angelenkt und auf unterschiedliche Längsachsenneigungen einstellbar. Zur Durchmischung und Homogenisierung besitzt der Feststoffreaktor jeweils zwei unter dem Tisch 17 und zwei oberhalb des Tisches 17 angeordnete Schneckenwellenpaare von gegenläufigen Schneckenwellen 161, 162 bzw. 163, 164, wie dies aus Fig. 3 und 4 ersichtlich ist. Die Vorrichtung besitzt ferner noch eine Notklappe 23, um bei Verstopfungen im betreffenden darunterliegenden Raum eingreifen zu können. Zur Entwässerung, auch während der Kreislaufführung des Bioabfalles, dient ein Siebblech 24 mit einem Gewebefilter als Abdeckung, wodurch das kohlenstoffbelastete Wasser über die Öffnung 25 abgezogen werden kann. Die beiden unteren Förderschnecken 161 und 162, die zur Förderung des Materiales in einer Richtung gegenläufig arbeiten, besitzen eine erste Förderwendel 26 im unteren Bereich und eine Förderwendel 27 im oberen Bereich mit größerer Steigung. Hierdurch wird erreicht, daß das unterhalb des Tischbleches 17 nach oben geförderte Material aufgrund der sich aus der höheren Steigung ergebenden höheren Fördergeschwindigkeit aufgelockert wird. Das über den Stutzen 28 dem Feststoff-Reaktor zugeleitete Wasser wird im Bereich der Förderwendel 27 mit größerer Steigung oder oberhalb des Tisches aufgegeben. Das Wasser dient zur Befeuchtung, sowohl um bereits abgezogenes Wasser zu ersetzen als auch um den jeweils gewünschten Feuchtegrad einstellen zu können, damit hinreichend Flüssigkeit zur Kohlenstoffaufnahme vorhanden ist. Oberhalb der Tischbleche 17 ist ebenfalls ein Förderschneckenpaar 163 und 164 angeordnet, um das von dem unter dem Tischblech liegenden Förderschneckenpaar nach oben geförderte Material nach unten zu transportieren. Durch diese obere Förderschnecke bzw. das Förderschneckenpaar wird vermieden, daß sich Materialbrücken bilden. Die jeweiligen Längsachsen der Förderschnecken liegen zueinander und zu den Tischblechen parallel. In den Tischblechen 17 befinden sich Rampen 28, an deren im wesentlichen senkrecht zur Tischblechebene stehenden Rückwand 29 Düsen 30 zur Luftzufuhr angeordnet sind. Die Belüftung des im Kreislauf geführten Materiales wird somit auch in dem Bereich vorgenommen, in dem das Material weitgehend aufgelockert und befeuchtet ist.

Nach mehrstündigem Umlauf des Bio-Materials wird zum Abpressen ein Schieber 31 in den unterhalb des Tischbleches liegenden Förderbereich gefahren, so daß die Föderwendel 26 nunmehr als Preßorgan dient, welches das Material gegen den verschlossenen Schieber 31 führt. Der Abpreßdruck wird indirekt gemessen, nämlich durch die Messung der Stromaufnahme am Förderschneckenmotor. Bei Erreichen eines vorgegebenen Sollwertes, der einen bestimmten Abpreßgrad entspricht, wird der Schieber wieder herausgefahren, wobei das Material weiter nach oben gefördert wird. Das abgepreßte Material wird über eine wiederverschließbare Öffnung 32 aus dem Feststoff-Reaktor 01 entleert bzw. abgeworfen.

Alternativ zu der in den Fig. 2 bis 6 beschriebenen einteiligen Vorrichtung ist es auch möglich, mehrere Feststoff-Reaktoren zum Mischen, Homogenisieren und Hygienisieren nebeneinander zu betreiben und nach dem Homogenisierungs- und Hygienisierungsvorgang das fertig behandelte Material in eine getrennte Preßvorrichtung 33 zu geben, die an den Auswurfschacht 32, der mit einem wiederverschließbaren Schieber 34 versehen ist, möglichst abschließend anzudocken. Bei dieser Ausführungsform entfällt der Schieber 31 als Teil der Preßvorrichtung gemäß der Vorrichtung in Fig. 2. Im übrigen ist der Feststoff-Reaktor entsprechend wie in Fig. 2 aufgebaut. Die Preßvorrichtung selbst besitzt eine Förderschnecke 35, die das eingefüllte Material gegen eine feste Wand fördert, verdichtet und damit abpreßt. Über geeignete Ablauflochbleche und Tuchfilter wird das abgepreßte Fluid entnommen. Vorzugsweise ist die Preßvorrichtung 33 auf Schienen verfahrbar ausgestattet, wobei der Schienenstrang so angelegt ist, daß sukzessive die Vorrichtung unterhalb einer jeden Mischvorrichtung bzw. dessen Abwurföffnung 32 gefahren und dort angeschlossen werden kann. Das Aufnahmevermögen der Preßvorrichtung entspricht im wesentlichen der Kapazität der Homogenisierungs- und Mischvorrichtung 01.

## Patentansprüche

1. Verfahren zur Behandlung von organischen Bio-Reststoffen, insbesondere aus kommunalen und/oder gewerblichen Abfallstoffen einschließlich roher und/oder gekochter Speisereste, landwirtschaftlicher Abfälle, insbesondere Tierexkremente und/oder pflanzlicher Bestandteile, die unter intensivem Mischen und Homogenisieren durch Pressen in eine flüssige Fraktion mit in Wasser gelösten oder lösbaren C-haltigen organischen Bestandteilen (DOC) und in aus den verbleibenden Feststoffen bestehenden festen Fraktionen getrennt werden,
**dadurch gekennzeichnet**,
daß die organischen Bio-Reststoffe vor dem Pressen über oberhalb und unterhalb eines schrägliegenden Tisches oder Tischblechen (17) jeweils parallel hierzu angeordneten Förderschnecken (16) in einem geschlossenen Kreislauf geführt werden, in dessen Verlauf die Bio-Reststoffe aufgelockert und die aufgelockerten Bio-Reststoffe befeuchtet und/oder belüftet werden, wobei die Auflockerung der Bio-Reststoffe durch eine Erhöhung der Fördergeschwindigkeit in einem Teil des Kreislaufes durchgeführt wird.

2. Verfahren nach Anspruch 1, dadurch gekennzeichnet, daß die Fördergeschwindigkeiten über das Steigungsmaß mindestens einer Förderschnecke eingestellt wird und/oder daß die Bio-Reststoffe über eine oder mehrere unterhalb des Tisches angeordnete Schnecken mit über ihren Längen unterschiedlichen Wendelsteigungen und nach oben zunehmendem Steigungsmaß geführt werden, wobei vorzugsweise das Steigungsmaß über die Länge der Schnecke(n) progressiv geändert wird.

3. Verfahren nach einem der Ansprüche 1 oder 2, dadurch gekennzeichnet, daß die Belüftung der im Kreislauf geführten Bio-Reststoffe über in Förderrichtung eingeblasene Luft erfolgt und/oder daß die Ausgangsfeuchte oder die Feuchte von 70 bis 80 Vol.-%, zumindest jedoch 40 bis 60 Vol.-%, während des Auswaschens von in Wasser gelösten organischen Bestandteilen, ggf. durch Zuführen oder Abführen, vorzugsweise durch Absaugung, von Wasser aufrechterhalten wird, wobei vorzugsweise das durch erfolgte Methanisierung von C-haltigen Bestandteilen befreite Wasser aus einem Anaerob-Reaktor rezyklierend zur Befeuchtung der Bio-Reststoffe verwendet wird.

4. Verfahren nach einem der Ansprüche 1 bis 3, dadurch gekennzeichnet, daß die im Kreislauf geführten Bio-Reststoffe nach ihrer Mischung und Homogenisierung in demselben Behälter durch Zuschaltung eines Preßorganes oder nach Abgabe in eine separate Preßeinrichtung abgepreßt werden, wobei vorzugsweise die Mischung und Homogenisierung der Bio-Reststoffe parallel in mehreren Behältern durchgeführt wird und zum Abpressen der Bio-Reststoffe eine (einzige) Preßvorrichtung, die unter den Behälter gefahren oder an ihm angedockt wird, verwendet wird.

5. Verfahren nach einem der Ansprüche 1 bis 4, dadurch gekennzeichnet, daß eine Temperatur von bis zu 70°C konstant aufrechterhalten wird, ggf. mit der anschließenden Maßnahme, abschließend Wärme zur Erzeugung einer Temperatur von 80°C bis 90°C zuzuführen, um eine möglichst vollständige Hygienisierung durchzuführen und/oder daß die bei den exotherm verlaufenden Reaktionen entstehende Wärme abgezogen und in den Anaerob-Reaktor geleitet wird.

6. Verfahren nach einem der Ansprüche 1 bis 5, dadurch gekennzeichnet, daß erst nach Unterschreiten eines vorgebbaren Gehaltes an gelösten C-haltigen Bestandteilen in der Flüssigkeit die verbleibenden Reststoffe auf einen möglichst niedrigen Restfeuchtigkeitsgehalt abgepreßt werden, bevor die gesamte Charge an Reststoffen abgeführt und anschließend kompostiert wird und/oder daß die als Suspension vorliegende C-haltige Bestandteile enthaltende Flüssigkeit sedimentiert wird, bevor sie einem Anaerob-Reaktor zugeführt wird, wobei vorzugsweise der beim Sedimentieren als Schlamm anfallende Feststoff als Animpfmaterial zur Reaktionsbeschleunigung, weiterhin vorzugsweise nach Sauerstoffanreicherung nach frischer Füllung den Bio-Reststoffen zugeführt wird.

7. Verfahren nach einem der Ansprüche 1 bis 6, dadurch gekennzeichnet, daß im Anaerob-Reaktor bei der Behandlung mit mesophilen Bakterien eine Temperatur von 45 bis 40°C, bei der Behandlung mit thermophilen Bakterien von 50 bis 55°C aufrechterhalten wird.

8. Vorrichtung zur Mischung und Homogenisierung von Bio-Reststoffen vor dem Abpressen der Flüssigfraktion von der festen Fraktion, mit einem mindestens zwei parallele Förderschnecken (16) enthaltenden Behälter (01), unter Bildung eines geschlossenen Förderkreislaufes, dadurch gekennzeichnet, daß oberhalb und unterhalb eines schrägliegenden Tisches oder Tischblechen (17) jeweils parallel hierzu Förderschnecken (161, 162; 163, 164) angeordnet sind, wobei die untere(n) Förderschnecke(n) (161, 162) das Material aufwärts- und die obere(n) Förderschnecke(n) (163, 164) das Material abwärtsfördern.

9. Vorrichtung nach Anspruch 8, dadurch gekennzeichnet, daß die obere(n) Förderschnecke(n) (163, 164) mit geringerer Fördergeschwindigkeit laufen als die untere(n) Förderschnecke(n) (161, 162) und/oder daß zumindest eine der Schnecken (161, 162), vorzugsweise die untere(n) Schnecke(n) (161, 162) unterschiedliche Steigungen der Schneckenwendel (26, 27) aufweisen, vorzugsweise mit einer progressive Änderung des Schneckenwendel-Steigungsmaßes.

10. Vorrichtung nach einem der Ansprüche 8 oder 9, dadurch gekennzeichnet, daß zum Abpressen der Bio-Reststoffe ein Schieber (31) in den Förderweg bewegbar ist, gegen den das Material zum Abpressen gefahren wird, wobei vorzugsweise der Öffnungsgrad des Schiebers (31), nämlich das Querschnittsmaß des blockierten Förderweges, regelbar ist und/oder die abgepreßte flüssige Fraktion über Lochbleche (24), die vorzugsweise mit Gewebedraht versehen sind, abführbar ist.

11. Vorrichtung nach einem der Ansprüche 8 bis 10, dadurch gekennzeichnet, daß in verschiedenen Förderbereichen unterschiedliche Drücke einstellbar sind und/oder daß im Bereich eines größeres Förderschneckensteigungsmaßes eine Flüssigkeitseingabevorrichtung vorgesehen ist.

12. Vorrichtung nach einem der Ansprüche 8 bis 11, dadurch gekennzeichnet, daß in Förderrichtung gerichtete Düsen (30) zur Luftzufuhr, vorzugsweise oberhalb des Tisches oder Tischbleches (17) vorgesehen sind und/oder im Tisch oder Tischblech (17) in Förderrichtung ansteigende Rampen (28) vorgesehen sind, an deren vorzugsweise in bezug auf die Tischblechebene vertikaler Rückseite (29) die Düsenaustrittöffnungen (30) angeordnet sind.

13. Vorrichtung nach einem der Ansprüche 8, 9, 11 oder 12, dadurch gekennzeichnet, daß mehrere Reaktoren zum Mischen und Homogenisieren der Bio-Reststoffe vorgesehen sind, die in eine separate, vorzugsweise einzige Preßvorrichtung (33) entladbar sind, wobei vorzugsweise die Preßvorrichtung auf Schienen oder Rädern verfahrbar ist und/oder die Preßvorrichtung ein oder mehrere Schneckenpressen (35) enthält.

14. Vorrichtung nach einem der Ansprüche 8 bis 13, dadurch gekennzeichnet, daß der Abpreßgrad über einen mit der Schnecke (26, 35) verbundenen Stromaufnehmer gemessen wird.

15. Vorrichtung nach einem der Ansprüche 8 bis 14, dadurch gekennzeichnet, daß der Stellwinkel des Behälters (01) zum Mischen und Homogenisieren veränderbar ist.

## Claims

1. Process for treating organic biological residues, in particular from municipal and/or industrial waste including raw and/or boiled remains of food, agricultural waste, in particular animal excrements and/or plant components, while being intensively mixed and homogenised being separated in a liquid fraction having organic carbon-containing components (DOC) dissolved or soluble in water and in solid fractions comprising of the remaining solid materials by pressing characterised in that the organic biological residues are conveyed before pressing above and below an inclined table or table-plates (17) by worm conveyors (16) arranged parallelly to the table or table plates in a closed loop, the biological residues being loosened up and moistened and/or air-ventilated, whereby the loosening up of the biological residues is executed by increasing the conveying velocity in a part of the closed loop.

2. Process according to claim 1, characterised in that the conveying velocities are adjusted by the lead size of at least one worm conveyor and/or that the biological residues are conveyed by one or more worms arranged below the table and having along their length different helical leads and an upward increasing lead size, whereby preferably the lead size changes progressively along the length of the worm(s).

3. Process according to one of the claims 1 or 2, characterized in that the air ventilation of the biological residues conveyed in a closed loop is executed by blowing air in conveying direction and/or that the initial moisture or the moisture of 70 to 80 Vol.%, but at least 40 to 60 Vol.%, is maintained while washing out the organic components dissolved in water, optionally by addition or removal, preferably by suction, whereby preferably for moistening the water is used which is recycled out of the anaerobe reactor and which is freed from carbon-containing components by methanisation.

4. Process according to one of the claims 1 to 3, characterised in that the biological residues conveyed in a closed loop are pressed by addition of pressing means after their mixing and homogenising in the same container or are pressed in a separate pressing device, whereby preferably mixing and homogenising of the biological residues is executed parallelly in several containers and for pressing of the biological residues one (single) pressing device is used which is disposed below the container or docked thereto.

5. Process according to one of the claims 1 to 4, characterised in that a constant temperature of up to 70°C is maintained, if necessary with the following treatment to add finally heat for generating a temperature of 80°C to 90°C to produce the best possible fully homogenisation and /or that the heat generated in the exothermic reaction is withdrawn and guided into the anaerobe reactor.

6. Process according to one of the claims 1 to 5, characterised in that the remaining residues are pressed onto a lowest possible moisture content not before an alleged content of dissolved carbon-containing components is achieved, before the whole charge of remaining materials is removed and then composted and/or that the fluid containing carbon components in form of a suspension is sedimented, before the fluid is conveyed to an anaerobe reactor, whereby preferably the solid material as a slurry coming from the sedimentation is conveyed to the biological residues as a vaccination material for accelerating the reactions, furthermore preferably after oxygen enrichment after fresh filling.

7. Process according to one of the claims 1 to 6, characterised in that in the anaerobe-reactor a temperature of 45°C to 40°C at the treatment with mesophile bacteria, a temperature of 50 to 55°C at the treatment with thermophile bacteria is maintained.

8. Device for mixing and homogenising of biological residues before separating the fluid fraction from the solid fraction by pressing, having at least two parallel worm conveyors (16) in a container (01), forming a closed conveying loop, characterised in that above and below an inclined table or table plates (17) worm conveyors (161, 162, 163, 164) are arranged parallelly thereto, whereby the lower worm conveyor(s) (161, 162) convey the material upward and the upper worm conveyor(s) convey the material downward.

9. Device according to claim 8, characterised in that the upper worm conveyor(s) (163, 164) run with lower conveyors velocities than the lower worm conveyor(s) (161, 162), preferably the lower worm(s) (161, 162) have different leads of the worm helix (26, 27), preferably with a progressive changing of the leadsize of the worm helix.

10. Device according to one of the claims 8 or 9, characterised in that for pressing the biological residues a slide plate (31) can be disposed into the conveying path, against which the material is conveyed for pressing, whereby preferably the grade of aperture of the slide plate (31), namely the cross-section size of the conveying path blocked up is adjustable and/or the pressed fluid fraction can be conveyed over meshed plates (24) which are preferably provided with wire netting.

11. Device according to one of the claims 8 to 10, characterised in that different pressures in different conveying regions are adjustable and/or that in the region of the greatest leadsize of the worm conveyor a fluid supply device is provided.

12. Device according to one of the claims 8 to 11, characterised in that nozzles (30) for air supply aligned in conveying direction are provided, preferably above the table or table plates (17) and/or there are provided ramps (28) ascending in the conveying direction and having nozzle outlet aperture at their back side which is preferably arranged vertically to the plane of the table plates.

13. Device according to one of the claims 8, 9, 11 or 12, characterised in that for mixing and homogenising of the biological residues several reactors are provided which can be disloaded into a separate preferably sole pressing device (33), whereby preferably the pressing device can be driven on rails or wheels and/or the pressing device has one or more worm conveyors (35).

14. Device according to one of the claims 8 to 13, characterised in that the grade of pressing can be measured by a current detector connected to the worm (26, 35).

15. Device according to one of the claims 8 to 14, characterised in that the angle of inclination of the container (01) for mixing and homogenising can be adjusted.

## Revendications

1. Procédé de traitement de résidus biologiques organiques, en particulier de déchets communaux et/ou industriels y compris des restes crus et/ou cuits de repas, des déchets agricoles, en particulier excréments animaux et/ou composants végétaux, qui, tout en mélangeant et homogénéisant de manière intensive, sont séparés par pressage en une fraction liquide avec des composants organiques contenant C dissous ou solubles dans l'eau (DOC), et dans des fractions solides se composant des matières solides restantes,
**caractérisé par le fait**
que, avant le pressage, les résidus biologiques organiques sont menés en circuit fermé par l'intermédiaire d'hélices transporteuses (16) disposées au-dessus et au-dessous d'une table ou de plaques de table (17) inclinée(s) et respectivement parallèlement à cela; au cours du circuit fermé, les résidus biologiques sont ameublis et les résidus biologiques ameublis sont humectés et/ou aérés, l'ameublissement des résidus biologiques étant effectué par une augmentation de la vitesse de transport dans une partie du circuit.

2. Procédé selon la revendication 1, caractérisé par le fait que les vitesses de transport sont réglées par la mesure du pas d'une hélice transporteuse du moins et/ou que les résidus biologiques sont menés par le biais d'une ou de pluieurs vis sans fin disposées au-dessous de la table, ayant des pas hélicoïdaux différents sur leur longueur et une mesure de pas croissante vers le haut, de préférence, la mesure du pas étant modifiée progressivement sur la longueur de la vis (des vis) sans fin.

3. Procédé selon l'une des revendications 1 ou 2, caractérisé par le fait que l'aération des résidus biologiques menés en circuit est effectuée par l'intermédiaire de l'air insufflé dans la direction de transport et/ou que l'humidité initiale ou l'humidité comprise entre 70 et 80 pourcent volumétrique, cependant, au moins entre 40 et 60 pourcent volumétrique est maintenue durant l'enlèvement par lavage de composants organiques dissous dans l'eau, le cas échéant par admission ou évacuation, de préférence par aspiration, d'eau, de préférence, l'au d'un réacteur anaérobie, libérée de composants contenant C au moyen de la méthanisation effectuée étant utilisée de façon recyclée pour humecter les résidus biologiques.

4. Procédé selon l'une des revendications 1 à 3, caractérisé par le fait que, après les avoir mélangés et homogénéisé, les résidus biologiques menés en circuit sont pressurés dans le même récipient en "mettant en circuit" un orgrane à presser ou après les avoir mis dans un dispositif séparé à presser, de préférence, le mélange et l'homogénéisation des résidus biologiques étant effectué parallèlement dans plusieurs récipients, et un (seul) dispositif à presser étant utilisé pour le pressurage des résidus biologiques, qui est placé sous le récipient ou accosté à celui-ci.

5. Procédé selon l'une des revendications 1 à 4, caractérisé par le fait qu'une température allant jusqu'a 70°C est maintenue de manière constante, le cas échéant, avec la mesure subséquente d'amener finalement de la chaleur pour générer une température comprise entre 80°C et 90°C afin de réaliser une hygiénisation aussi complète que possible, et/ou que la chaleur produite lors des réactions se déroulant de façon exothermique est évacuée et conduite dans le réacteur anaérobie.

6. Procédé selon l'une des revendications 1 à 5, caractérisé par le fait que ce n'est qu'après être inférieur à une teneur prédéterminable en composants dissous contenant C dans le liquide que les résidus restants sont pressurées de manière à atteindre une teneur restante en humidité aussi faible que possible, avant que l'ensemble de la charge de résidus soit évacué et par la suite composté, et/ou que le liquide présent sous forme de suspension et contenant des composants contenant C est déposé par sédimentation avant qu'il soit amené à un réacteur anaérobie, de préférence, la matière solide résultant en tant que boue durant la sédimentation étant amenée aux résidus biologiques comme agent d'inoculation pour accélérer la réaction, en outre de préférence après l'enrichissement en oxygène après un remplissage frais.

7. Procédé selon l'une des revendications 1 à 6, caractérisé par le fait que l'on maintient, dans le réacteur anaérobie, une température comprise entre 45 et 40°C durant le traitement avec des bactéries mésophiles, et une température comprise entre 50 et 55°C lors du traitement avec des bactéries thermophiles.

8. Dispositif de mélange et d'homogénéisation de résidus biologiques avant d'extraire par pressurage la fraction liquide de la fraction solide, avec un récipient (01) contenant du moins deux hélices transporteuses (16) parallèles, formant un circuit fermé de transport, caractérisé par le fait que des hélices transporteuses (161, 162; 163, 164) sont disposées au-dessus et au-dessous d'une table ou de plaques de table inclinée(s) (17), respectivement parallèlement à cela, l'hélice (les hélices) transporteuse(s) inférieure(s) (161, 162) transportant la matière vers le haut et l'hélice (les hélices) transporteuse(s) supérieure(s) (163, 164) transportant la matière vers le bas.

9. Dispositif selon la revendication 8, caractérisé par le fait que l'hélice (les hélices) transporteuse(s) supérieure(s) (163, 164) marchent à une vitesse de transport plus faible que l'hélice (les hélices) transporteuse(s) inférieure(s) (161, 162), et/ou que l'une au moins des vis sans fin (161, 162), de préférence la (les) vis sans fin inférieure(s) (161, 162) présentant des pas différents de l'hélice (26, 27) de la vis sans fin, de préférence avec une modification progressive de la mesure de pas de l'hélice de la vis sans fin.

10. Dispositif selon l'une des revendications 8 ou 9, caractérisé par le fait que, pour le pressurage des résidus biologiques, un coulisseau (31) peut être déplacé dans le chemin de transport, contre lequel la matière est transportée pour être pressurée, de préférence, le degré d'ouverture du coulisseau (31), à savoir la mesure de la section du chemin bloqué de transport, étant réglable et/ou la fraction liquide extraite par pressurage pouvant être évacuée par des tôles perforées (24) qui, de préférence, sont pourvues de treillis de fil métallique.

11. Dispositif selon l'une des revendications 8 à 10, caractérisé par le fait que l'on peut régler des pressions différentes dans des zones de transport différentes, et/ou qu'un dispositif à introduire du liquide est prévu dans la zone d'une plus grande mesure de pas de l'hélice transporteuse.

12. Dispositif selon l'une des revendications 8 à 11, caractérisé par le fait que des tuyères (30) pour l'amenée d'air, dirigées dans la direction de transport sont prévues, de préférence au-dessus de la table ou de la plaque de table (17), et/ou que des rampes (28) montant dans la direction de transport sont prévues dans la table ou la plaque de table (17), dont la surface arrière (29) qui, de préférence, est disposée verticalement par rapport au plan de la plaque de table, présente les orifices de sortie (30) de tuyère.

13. Dispositif selon l'une des revendications 8, 9, 11 ou 12, caractérisé par le fait que l'on prévoit plusieurs réacteurs pour mélanger et l'homogénéiser les résidus biologiques, qui peuvent être déchargés dans un dispositif à presser (33) séparé, de préférence unique, de préférence, le dispositif à presser étant déplaçable sur des rails ou roues, et/ou le dispositif à presser contenant plusieurs presses à vis (35).

14. Dispositif selon l'une des revendications 8 à 13, caractérisé par le fait que le degré de pressurage est mesuré par l'intermédiaire d'un détecteur de courant connecté à la vis sans fin (26, 35).

15. Dispositif selon l'une des revendications 8 à 14, caractérisé par le fait que l'angle d'inclinaison du récipient (01) pour mélanger et homogénéiser peut être modifié.
